# EUROPEAN PATENT SPECIFICATION

(11) **EP 3 589 743 B1**
(45) Date of publication and mention of the grant of the patent: **21.06.2023**
(21) Application number: 18709886.8
(22) Date of filing: 23.02.2018
(51) Int. Cl.: C12Q 1/22, C12M 1/12

(54) **RECOVERY MEDIUM FOR DETECTION OF MICROORGANISMS BY FLUORESCENCE**
RÜCKGEWINNUNGSMEDIUM ZUM NACHWEIS VON MIKROORGANISMEN DURCH FLUORESZENZ
MILIEU DE RÉCUPÉRATION POUR LA DÉTECTION DE MICRO-ORGANISMES PAR FLUORESCENCE

(30) Priority: 28.02.2017 US 201762464607 P
(43) Date of publication of application: 08.01.2020
(73) Proprietor: American Sterilizer Company, Mentor, OH 44060-1834 (US)
(72) Inventor: SOTO, Kelly, Madison, Ohio 44057 (US); WHITE, Nicholas, Mentor, Ohio 44060 (US); AUFMUTH, Jennifer, Mentor, Ohio 44060 (US); SMITH, Sarah, Painesville, Ohio 44077 (US); FIX, Kathleen, A., Willoughby, Ohio 44094 (US); YIRAVA, William, A., Willoughby, Ohio 44094 (US)
(74) Representative: Ström & Gulliksson AB
(86) International application number: PCT/US2018/019372
(87) International publication number: WO 2018/160449

(56) References cited:
- US-A1- 2008 070 231
- US-A1- 2014 262 829
- US-A1- 2014 273 072
- US-A1- 2016 102 335
- US-B2- 8 173 388

## Description

### Technical Field

The present application relates to a method and device for detecting viable microorganisms by fluorescence, in particular, for evaluating the effectiveness of sterilization processes. More particularly, the present invention relates to a recovery medium for a biological indicator.

### Background

Biological indicators, which typically comprise test microorganisms (e.g., spores), are used for evaluating the efficacy of sterilization processes. The biological indicator is placed in a sterilization chamber and subjected to a sterilization process along with the load intended for sterilization (e.g., a medical device). Upon completion of the sterilization process, the biological indicator is exposed to a liquid growth support medium under conditions that would promote the growth of any surviving test organism cells. A successful sterilization process is indicated by a complete inactivation (no outgrowth) of the test microorganisms. An unsuccessful sterilization process is indicated by an incomplete inactivation (outgrowth detected) of the test microorganisms. The recovery medium may contain a fluorogenic substrate which, on contact with enzymes synthesized by microorganisms, are cleaved and become fluorescent. Detection of biological activity may be carried out, for example, by a fluorometer.

### Summary

Biological indicators used in sterility assurance may contain a recovery medium that provides for cultivation of viable test organisms. The typical recovery medium for aerobic bacteria is Tryptic Soy Broth (TSB), which is a complex, general purpose broth commonly used in microbiological laboratories. TSB has been the starting point for media for numerous commercial biological indicators for several sterilization methods, including steam, vapor phase hydrogen peroxide, ethylene oxide, etc. These broth formulations may contain dyes which respond to pH, thereby providing visual evidence of the growth of organisms, or which respond to enzymatic activity, producing a fluorescent moiety which is read by a fluorometer.

Biological indicators are used to provide assurance that items subjected to a sterilization process have been rendered sterile at the end of a successful cycle. Biological indicators for sterilization processes make use of the "Most Resistant Organism" (MRO) for the process. This is generally a spore-forming organism, as spores are a very difficult to kill survival state of the organism. In the case of steam and vapor phase hydrogen peroxide sterilization, the organism is typically the bacterium *Geobacillus stearothermophilus*, which forms spores when subjected to deficient conditions.

An aspect of biological indicators that has seen continued efforts for improvement is the time required to determine a negative response. A negative response indicates a successful sterilization cycle. For the purposes of submitting a new biological indicator to the Food and Drug Administration for approval, the standard incubation time to determine a negative growth response is considered to be seven days. Any time less than seven days is considered a Reduced Incubation Time (RIT).

With the advent of more complicated medical devices and procedures and the need to maintain low levels of inventory to attain reduced operating costs, medical clinics and hospitals do not wish to wait seven days to determine if a load of medical instruments is sterile. Therefore, biological indicators (BIs) with shorter RITs have been tested and approved for use by the FDA. Common RITs for a visual color change BI is several hours to a day or two. The visual color change BI requires no special equipment other than a unit to keep the BI at the desired temperature.

Because measurement of fluorescent molecules is more sensitive than colorimetric methods, an advance was made with the first fluorescent BIs, where the RIT is 30 minutes to 4 hours. The fluorescent BI requires a unit capable of maintaining temperature, delivering light of a specified wavelength, and the detection of light generated at another wavelength. Reader/incubators approved by the FDA also contain some means of determining the difference between a positive response (non-sterile) and a negative response (successful sterilization). The positive response can be expressed more quickly by the reader/incubator than a negative response. Therefore, notification of a non-sterile result can occur more quickly than can the assurance of a sterile result.

TSB is a complex medium containing two primary components of less defined characteristics. The first is tryptone, which is a digest of casein by protease, resulting in a mixture of peptides. The second is phytone, an enzymatic digest of soybean meal. TSB also contains sodium chloride (an osmotic mediator), dipotassium phosphate (a buffer), and glucose (a reducing sugar and food source).

The first two components are natural products and, while the process is controlled, the constituents of the natural product may change based upon feedstocks. The origin of the casein and/or soybean may induce changes due to climactic conditions of growth, variations of growth for animal or plant, nutritional variations, etc. Therefore, the final products cannot be well-defined and, while appropriate for a general purpose medium, will add variance in a sensitive detection method which uses fluorescence.

Of particular significance with TSB is the fact that the media components are a yellow-brown in color and, when mixed with a reducing sugar, are subject to the Maillard reaction. The Maillard reaction encompasses non-enzymatic browning that occurs when a mixture of amino acids and reducing sugars are heated. This browning reaction is recognizable, for example, from the brown color of baked goods and sear marks on cooked meats. The Maillard reaction has also been found to occur at ambient temperature.

Therefore, a TSB medium will be yellow-brown initially and will continue to increase in color with subsequent steam sterilization exposures and/or aging. This is evidenced by measuring the absorbance of a solution at a wavelength of 420 nm. In addition, the Maillard reaction also produces a mixture of fluorescent compounds, some reported with emission spectra in the 420 to 440 nm wavelength range. This background fluorescence can adversely affect the accuracy and speed at which the enzyme activity of the biological indicator is detected.

A common fluorescent indicator family, the methylumbelliferyl pyranosides (also known as hydroxy methyl coumarins), emits a fluorescent signal in the 445 nm region. Methylumbelliferyl gluco- or galacto- pyranoside is used in the determination of glucosidases or galactosidases (enzymes). When the appropriate enzyme acts on the fluorescent precursor, the molecule is cleaved, releasing either glucose or galactose and the fluorescent compound, 4-methylumbellliferone (4MU). 4MU is excited at 365nm in a glycine buffer and emits at 445nm.

The presence of both the colored components in the TSB media and the fluorescent compounds generated by the Maillard reaction will interfere with or increase variation in the output signal of interest, for example, emitted light at about 445 nm.

In one aspect of the present invention there is provided a defined recovery medium that is clear in color and does not contain the combination of compounds that participate in the Maillard reaction. The recovery media described herein are particularly suited for following enzymatic reactions where the fluorescent product is excited at a wavelength of 320 to 370 nm and emits at a wavelength of 420 to 460 nm.

While use of the recovery media is described herein as being particularly suitable as a recovery medium for a self-contained biological indicator system, the recovery media described herein may also be used for determining an enzyme concentration or to follow an enzymatic reaction through the use of a fluorescent moiety. The recovery media may also be used to produce a calibration standard for the reader/incubators mentioned above.

In an aspect of the present invention a recovery medium for detecting the presence of microorganisms includes at least one mineral salt; and at least one fluorogenic substrate; wherein the recovery medium is clear in color and is substantially free of reducing sugar. The recovery medium may further include at least one amino acid and/or at least one vitamin.

The fluorogenic substrate of the recovery medium may include a 4-methylumbelliferyl derivative. The 4-methylumbelliferyl derivative may include 4-methylumbelliferyl α-D-glucopyranoside.

In one embodiment, the fluorogenic substrate of the recovery medium includes a cleavable fluorophore group having a fluorescence emission in the range of 420 nm to 460 nm for an excitation wavelength in the range of 320 nm to 370 nm. The cleavable fluorophore may include 4-methylumbelliferone (4-MU).

In one embodiment, the recovery medium does not absorb light energy in the wavelength range of 420 nm to 450 nm.

In one aspect of the invention there is provided a method of determining the effectiveness of a sterilization procedure, the method including the steps of: (i) placing a biological indicator containing at least one active enzyme into a sterilization chamber; (ii) performing the sterilization procedure within the chamber wherein the biological indicator is exposed to a sterilization medium; (iii) contacting the biological indicator with a recovery medium that includes at least one mineral salt, and at least one fluorogenic substrate comprising a cleavable fluorophore group, wherein the recovery medium is clear in color and is substantially free of reducing sugar; (iv) incubating the biological indicator for a period of time; and (v) detecting the presence or absence of a fluorescent signal emitted by the fluorophore group.

The sterilization medium of the sterilization procedure may include steam, dry heat, radiation, plasma, one or more gaseous sterilants and/or one or more liquid sterilants.

In another aspect of the invention there is provided a sterilization monitor that includes a first compartment containing a biological indicator, the biological indicator containing at least one active enzyme, the first compartment being adapted to permit the biological indicator to be brought into contact with a sterilization medium during a sterilization process; and a second compartment containing a recovery medium, the second compartment being adapted to maintain the recovery medium separate from the biological indicator during the sterilization process, and to permit the recovery medium to contact the biological indicator after the biological indicator has been exposed to the sterilization medium, wherein the recovery medium includes at least one mineral salt; and at least one fluorogenic substrate; wherein the recovery medium is clear in color and is substantially free of reducing sugar.

### Brief Description of the Drawings

Fig. 1 is a schematic illustration of a sterilization monitor suitable for use with the present invention, the sterilization monitor being shown in a pre-activated configuration.
Fig. 2 is a schematic illustration of the sterilization monitor of Fig. 1 in an activated configuration.
Fig. 3 is a schematic illustration of another embodiment of a sterilization monitor suitable for use with the present invention, the sterilization monitor being shown in pre-activated configuration.

### Detailed Description

The term "microorganism" refers to a microscopic living organism. The microorganisms may be unicellular, multicellular, or in the form of cell clusters. The microorganisms may comprise bacteria, archaea, protozoa, fungi, algae, viruses, multicellular animal parasites (helminths), or a combination of two or more thereof. The microorganisms may comprise spores. The microorganisms may comprise bacterial spores.

The term "bacteria" refers to a domain of prokaryotic microorganisms. The bacteria may be unicellular microorganisms. The cells may be described as prokaryotic because they lack a nucleus. The bacteria cells may have one of four major shapes: bacillus (rod shaped), coccus (spherical shape), spirilla (spiral shape), or vibrio (curved shape). The bacteria may have a peptidoglycan wall. The bacteria may divide by bacteria fission. The bacteria may possess flagella for motility. The bacteria may be classified as either Gram-positive or Gram-negative when using Gram staining. The bacteria may be divided based on their response to gaseous oxygen into the following groups: aerobic (living in the presence of oxygen), anaerobic (living without oxygen), and facultative anaerobic (can live in both environments). The bacteria may be classified as heterotrophs or autotrophs. Autotrophs make their own food by using the energy of sunlight or chemical reactions, in which case they are called chemoautotrophs. Heterotrophs obtain their energy by consuming other organisms. The bacteria that use decaying life forms as a source of energy may be called saprophytes.

The term "spore" refers to a unit of asexual reproduction that may be adapted for dispersal and survival for extended periods of time under unfavorable conditions. Spores are highly resistant, dormant cell types. Endospores (or simply spores) form within the vegetative mother cell in response to adverse changes in the environment, most commonly nutrient depletion. The mother cell undergoes an asymmetrical cell division, where it replicates its genetic material, which is then surrounded by multiple concentric and spore specific layers. The mother cell then disintegrates, releasing the mature dormant spore which requires neither nutrients, water nor air for survival and is protected against a variety of trauma, including extremes of temperature, radiation, and chemical assault.

The term "bacterial spore" refers to a spore produced by bacteria.

The term "test microorganism" refers to a microorganism that may be used to test the efficacy of a sterilization process. The test microorganism may be more resistant to a sterilization process than the organisms intended for destruction during the sterilization process. In theory, if the test microorganisms were to die during a sterilization process, then all organisms intended for destruction during the sterilization process that are less resistant to the sterilization than the test microorganisms would also die. The test microorganisms may comprise bacteria. The test microorganisms may comprise spores. The test microorganisms may comprise bacterial spores. The test microorganisms may comprise spores of the *Bacillus* or *Clostridia* genera. The test microorganisms may comprise spores of *Geobacillus stearothermophilus, Bacillus atrophaeus, Bacillus sphaericus, Bacillus anthracis, Bacillus pumilus, Bacillus coagulans, Clostridium sporogenes, Clostridium difficile, Clostridium botulinum, Bacillus subtilis globigii, Bacillus cereus, Bacillus circulans*, or a mixture of two or more thereof. The test microorganisms may comprise *Geobacillus stearothermophilus* spores, *Bacillus atrophaeus* spores, or a mixture thereof.

The term "biological indicator" refers to an article or a material that can be used to determine the efficacy of a sterilization process. The biological indicator may comprise test microorganisms (e.g., bacteria, spores or bacterial spores). The biological indicator may comprise test microorganisms on a carrier. The biological indicator may comprise bacteria, the bacteria may be present within a defined space or deposited on a carrier. The biological indicator may comprise spores (e.g., bacterial spores), the spores may be present within a defined space or on a carrier. The biological indicator may comprise a spore strip.

The term "sterilization" refers to rendering a substance incapable of reproduction, metabolism and/or growth. While this is often taken to mean total absence of living organisms, the term may be used herein to refer to a substance free from living organisms to a degree previously agreed to be acceptable. Unless otherwise indicated, the term sterilization is used herein to also refer to methods and procedures less rigorous than sterilization, for example, disinfection, sanitization, and the like. The biological indicator and the processes and apparatus described herein may be used in health care fields, scientific fields, and the like. These may be used in commercial and industrial applications where sterilization, disinfection, sanitization, decontamination, cleaning, and the like, may be desired. The commercial and industrial applications may include processes such as food processing, pasteurization, soil remediation, water remediation, and the like.

In one aspect of the present invention, a recovery medium for a biological indicator includes a liquid nutritive support in which at least one fluorogenic substrate is solubilized. The medium is clear in color, thereby increasing the efficiency of measured fluorescence and measurement sensitivity for fluorescent assays based upon methylumbelliferyl pyranoside precursors and/or any other assay which generates fluorescent molecules with emission wavelengths in the 400 to 450 nm range.

The term "clear in color" described above means that the medium is substantially colorless in the visible light region of about 390 to 700 nm.

The recovery medium includes a mixture of amino acids, vitamins, metal ions, mineral salts, and a buffer solution. The amino acid component may include mixtures of two or more of L-glutamic acid, L-histidine, L-isoleucine, L-methionine, and L-valine, and sodium and/or hydrochloride salts of the amino acids. Exemplary vitamins may be selected from among D-biotin, thiamine and nicotinic acid. The medium may contain one or more of iron chloride, calcium chloride, zinc sulfate, manganese chloride, ammonium chloride, sodium chloride, magnesium sulfate, and potassium and dipotassium phosphate as a base.

The recovery medium includes an enzyme substrate, which when acted upon by the indicator enzyme is converted into an enzyme-modified product. The enzyme substrate may comprise a fluorogenic substrate, defined herein as a material that is acted upon by an enzyme to produce a fluorescent compound.

There are two basic types of fluorogenic substrates that may be used for the detection of specific indicator enzymes. The first type of fluorogenic substrate may be given a chemical formula such as, AB. When acted upon by the indicator enzyme, AB, may break down to A+B. B, for example, may be fluorescent. In one embodiment, two B compounds may react together to produce the fluorescent signal. A specific example of a fluorogenic substrate of this type may be 4-methylumbelliferyl phosphate. In the presence of the indicator enzyme phosphatase, the substrate may be broken down into 4-methylumbelliferone and phosphate. Other fluorogenic substrates of this type may include the derivatives of 4-methylumbelliferyl, 7-amido-4-methylcoumarin, indoxyl and fluorescein.

The second type of fluorogenic substrate may be given by the chemical formula CD, for example, which may be converted by a specific enzyme to C+D. However, neither C nor D may be fluorescent, but D may be capable of being further reacted with compound Z to give a fluorescent compound, thus indicating enzyme activity. A specific fluorogenic example of this type may be the amino acid lysine. In the presence of the enzyme lysine decarboxylase, lysine may lose a molecule of CO₂. The remaining part of the lysine may then be called cadaverine, which is strongly basic. A basic indicator such as 4-methylumbelliferone may be incorporated and may be fluorescent in the presence of a strong base.

A number of enzyme substrates for indicator enzymes of diverse origins may be used. These may include fluorogenic 4-methylumbelliferyl derivatives (hydrolyzable to 4-methylumbelliferone (4-MU)); derivatives of 7-amido-4-methyl-coumarin; diacetylfluorescein derivatives; and fluorescamine.

Derivatives of 4-methylumbelliferyl that may be used as the enzyme substrate may include: 4-methylumbelliferyl-2-acetamido-4,6-O-benzylidene-2-deoxy-beta-D-lucopyranoside; 4-methylumbelliferyl acetate; 4-methylumbelliferyl-N-acetyl-beta-D-galactosaminide; 4-methylumbelliferyl-N-acetyl-alpha-D-glucosaminide; 4-methylumbelliferyl-N-acetyl-beta-D-glucosaminide; 2'-(4-methylumbelliferyl)-alpha-D-N-acetyl neuraminic acid; 4-methylumbelliferyl-alpha-L-arabinofuranoside; 4-methylumbelliferyl alpha-L-arabinoside; 4-methylumbelliferyl butyrate; 4-methylumbelliferyl-beta-D-cellobioside; methylumbelliferyl-beta-D-N, N'-diacetyl chitobioside; 4-methylumbelliferyl elaidate; 4-methylumbelliferyl-beta-D-fucoside; 4-methylumbelliferyl-alpha-L-fucoside; 4-methylum bell iferyl-beta-L -fucoside; 4-methylumbelliferyl-alpha-D-galactoside; 4-methylumbelliferyl-beta-D-galactoside; 4-trifluoromethylumbelliferyl beta-D-galactoside; 6,8-difluoro-4-methylumbelliferyl-beta-D-galactoside; 4-methylumbelliferyl-alpha-D-glucoside; 4-methylumbelliferyl-beta-D-glucoside; 4-methylumbelliferyl-7,6-sulfo-2-acetamido-2-deoxy-beta-D-glucoside; 4-methylumbelliferyl-beta-D-glucuronide; 6,8-difluor-4-methylumbelliferyl-beta-D-glucuronide; 4-methylumbelliferyl p-guanidinobenzoate; 4-methylumbelliferyl heptanoate; 4-m ethylum bell iferyl-alpha-D-m annopyranoside; 4-methylumbelliferyl-beta-D-mannopyranoside; 4-methylumbelliferyl oleate; 4-trifluoromethylumbelliferyl oleate; 4-methylumbelliferyl palmitate; 4-methylumbelliferyl phosphate; 4-methylumbelliferyl propionate; 4-methylumbelliferyl stearate; 4-methylumbelliferyl sulfate; 4-methylumbelliferyl-beta-D-N, N', N"-triacetylchitotriose; 4'-methylumbelliferyl 2,3,5-tri-beta-benzoyl-alpha-L-arabinofuranoside; 4-methylumbelliferyl-beta-trimethylammonium cinnamate chloride; 4-methylumbelliferyl 4-guanidinobenzoate; and 4-methylumbelliferyl-beta-D-xyloside.

Derivatives of 7-amido-4-methylcoumarin that may be used as the enzyme substrate may include: L-alanine-7-amido-4-methylcoumarin; L-proline-7-amido-4-methylcoumarin; L-tyrosine-7-amido-4-methylcoumarin; L-arginine-7-amido-4-methylcoumarin; L-citrulline-7-amido-4-methylcoumarin; L-leucine-7-amido-4-methylcoumarin; L-methionine-7-amido-4methylcoumarin; L-pyroglutamic acid 7-amido-4-methylcoumarin; L-aspartic acid beta-(7-amido-4-methylcoumarin); L-glutamic acid 1-(7-amido-4-methylcoumarin); L-phenylalanine-7-amido-4-methylcoumarin; and 7-glutaryl-phenylalanine-7-amido-4-methylcoumarin.

Peptide derivatives of 7-amido-4-methyl coumarin that may be used as the enzyme substrate may include: N-t-BOC-Ile-Glu-Gly-Arg 7-amido-4-methylcoumarin; N-t-BOC-Leu-Ser-Thr-Arg 7-amido-4-methylcoumarin; N-CBZ-Phe-Arg 7-amido-4-methylcoumarin; N-succinyl-Leu-Tyr-7-amido-4-methylcoumarin; Gly-Pro 7-amido-4-methylcoumarin; Pro-Phe-Arg 7-amido-4-methylcoumarin; N-t-BOC-Val-Pro-Arg 7-amido-4-methylcoumarin; and N-glutaryl-Gly-Arg 7-amido-4-methylcoumarin.

Derivatives of diacetylfluorescein that may be used as the enzyme substrate may include fluorescein diacetate, fluorescein dibutyrate, 2',7'-dichlorofluorescein diacetate, fluorescein di-(beta-D-N-acetygalactosamine), fluorescein di-(beta-D-galactoside), fluorescein mono-(beta-D-galactoside), and fluorescein dilaurate.

The enzyme substrate used may depend upon the identity of the indicator enzyme whose activity is under study. Where the indicator enzyme whose activity is to be detected is alpha-D-glucosidase, chymotrypsin or fatty acid esterase, a fluorogenic enzyme substrate that may be used may be 4-methylumbelliferyl-alpha-D-glucoside, 7-glutarylphenylalanine-7-amido-4-methyl coumarin, or 4-methylumbelliferyl heptanoate, respectively. Where the indicator enzyme whose activity is to be detected is alpha-L-arabinofuranosidase, a fluorogenic enzyme substrate that may be used may be 4-methylumbelliferyl-alpha-L-arabinofuranoside. Where the indicator enzyme whose activity is to be detected is beta-D-glucosidase, a fluorogenic enzyme substrate that may be used may be 4-methylumbelliferyl-beta-D-glucoside.

In one embodiment, the fluorescent precursor molecule is 4-methylumbelliferyl-α-D-glucopyranoside (MUD) in dimethylsulfoxide (DMSO). Enzyme cleavage releases 4-MU, resulting in fluorescence associated with the 4-MU anion having emission wavelength of 445 nm at an excitation wavelength of 365 nm. Other fluorescent precursor molecules dissolved in other solvents may be used.

An additional constituent of the recovery medium may be a non-reducing sugar. A non-reducing sugar is a carbohydrate that is not oxidized by a weak oxidizing agent (an oxidizing agent that oxidizes aldehydes, but not alcohols) in basic aqueous solutions. The characteristic property of non-reducing sugars is that, in basic aqueous medium, they do not generate any compounds containing an aldehyde group. Examples of non-reducing sugars include sucrose, trehalose, raffinose and melezitose. Non-reducing sugars, which do not participate in the Maillard reaction, are preferred as they do not impart a yellow brown color to the media upon interaction with amino acids. Additionally, they provide an energy/food source for the bacteria.

The pH of the recovery medium is preferably about 7.2 to 7.5 to allow conditions for microorganism metabolism. The pH could also vary from acid levels up to about pH 11. The media is clear in color and does not absorb light in the 420-450 nm region to the extent that TSB media does, thereby increasing the efficiency of measured fluorescence and measurement sensitivity.

The medium does not include a reducing sugar and, therefore, is not subject to the Maillard reaction. Reducing sugars include maltose, lactose, melibiose, cellobiose, gentiobiose, fructose, galactose, glucose, glyceraldehyde, ribose and xylose. The absence of a reducing sugar also reduces the possibility of extraneous fluorescent molecules being generated which could interfere, as a positive bias, with accurate determination of the enzyme of interest. As discussed above, the medium is not yellow brown in color and will, therefore, allow for an increase in enzyme detection sensitivity.

Even in the absence of a sugar source, the recovery media has been found to allow growth of organisms in shake flasks.

In an aspect of the present invention, the recovery medium contains the following constituents within the concentration ranges indicated in Table 1 below.

**TABLE 1**

| **Component** | **Molarity Range** | | | **g/L Range** | |
|---|---|---|---|---|---|
| | **Low** | **High** | | **Low** | **High** |
| **Amino Acids** | | | | | |
| L-Glutamic acid | 0.5 mM | 100 mM | | 0.08 | 16.91 |
| L-Histidine | 0.1 mM | 100 mM | | 0.016 | 15.5 |
| L-Isoleucine | 0.1 mM | 100 mM | | 0.014 | 13.1 |
| L-Methionine | 0.1 mM | 100 mM | | 0.014 | 14.9 |
| L-Valine | 0.1 mM | 100 mM | | 0.012 | 11.7 |
| | | | | | |

| **Vitamins** | | | | | |
|---|---|---|---|---|---|
| D-Biotin | 1 µM | 50 µM | | 0.00024 | 0.012 |
| Thiamine HCl | 1 µM | 50 µM | | 0.00026 | 0.013 |
| Nicotinic Acid | 1 µM | 50 µM | | 0.00012 | 0.0062 |
| | | | | | |

| **Metals/Minerals** | | | | | |
|---|---|---|---|---|---|
| FeCl₃ | 100 nM | 10 mM | | 0.000027 | 2.70 |
| CaCl₂ | 0.01 mM | 0.1M | | 0.0011 | 11.1 |
| ZnSO₄ | 0.1 µM | 100 µM | | 0.000029 | 0.029 |
| MnCl | 0.1 µM | 1 mM | | 0.000013 | 0.126 |
| NH₄Cl | 1 mM | 1 M | | 0.05 | 53.5 |
| NaCl | 0.5 mM | 0.5 M | | 0.03 | 29.22 |
| MgSO₄ | 0.01 mM | 0.1M | | 0.0012 | 12.04 |
| K₂HPO₄ | 1 mM | 0.5 M | | 0.17 | 87.1 |
| KH₂PO₄ | 1 mM | 0.5 M | | 0.136 | 68.04 |
| | | | | | |

| **Enzyme Substrate** | | | | | |
|---|---|---|---|---|---|
| MUD | 1 µM | 0.1 M | | 0.00034 | 33.8 |
| | | | | | |

| **Dissolution Aid or Solvent** | | | | | |
|---|---|---|---|---|---|
| DMSO | 0.0002% | 10% | | - | - |

The concentration of enzyme substrate in the recovery medium may be dependent upon the identity of the enzyme substrate and the indicator enzyme, the amount of enzyme-modified product that must be generated to be detectable, and the amount of time required to determine whether an indictor enzyme is present. The amount of enzyme substrate that may be sufficient may be the amount needed to react with any indicator enzyme that may be present after the sterilization has been completed such that an enzyme-modified product at a molar concentration of at least about 10⁻¹⁵ molar may be produced within a period of up to about 4 hours, or a molar concentration of at least about 10⁻⁸ molar within a period up to about 2 hours.

The recovery medium may be combined with the biological indicator after the biological indicator has been subjected to the sterilization cycle. The recovery medium containing the biological indicator can then be incubated. Incubation may be continued for a period of time and under conditions sufficient to liberate a detectable amount of the indicator enzyme, assuming any of the biological indicator remains functional. In general, the amount of indicator enzyme which may be detectable may be as low as about 1 × 10⁻¹⁵ molar. The incubation conditions may be sufficient to generate at least about 1 × 10⁻⁸ molar of indicator enzyme, or from about 1 × 10⁻⁶ to about 1 × 10⁻⁵ molar of indicator enzyme. The incubation time and temperature needed to produce a detectable amount of indicator enzyme may depend upon the identity of the indicator enzyme, and the concentration of the indicator enzyme in the recovery medium. The incubation temperature may be in the range from about 20°C to about 70°C. The incubation time may be in the range up to about 4 hours, or in the range from about 0.01 to about 4 hours, or in the range from about 0.01 to about 3 hours, or in the range from about 0.01 to about 2 hours, or in the range from about 0.01 to about 1 hour. The indicator enzyme acts upon the enzyme substrate to form an enzyme-modified product which can be detected. Detection can be achieved within a period of time of up to about 4 hours, or about 0.01 to about 4 hours, or about 0.01 to about 3 hours, or about 0.01 to about 2 hours, or about 0.01 to about 1 hour, or about 0.01 to about 0.7 hour, or about 0.01 to about 0.5 hour.

The biological indicator, although herein described primarily in terms of a single indicator enzyme, may provide a plurality of indicator enzymes. For example, the biological indicator may provide three types of indicator enzymes, one enzyme being resistant to heat, a second being resistant to gaseous sterilizing media, and a third being resistant to radiation, e.g., gamma or beta irradiation.

In one embodiment, the composition of the recovery medium is as shown in Table 2 below.

**Table 2**

| **Components** | g/L |
|---|---|
| **Amino Acids** | |
| L-Glutamic acid | 0.400 |
| L-Histidine | 0.042 |
| L-Isoleucine | 0.100 |
| L-Methionine | 0.052 |
| L-Valine | 0.126 |
| | |

| **Vitamins** | |
|---|---|
| D-Biotin | 0.001 |
| Thiamine HCl | 0.001 |
| Nicotinic Acid | 0.001 |
| | |

| **Metals/Minerals** | |
|---|---|
| FeCl₃ | 185 nM |
| CaCl₂ | 0.005 |
| ZnSO₄ | 0.005 |
| MnCl | 0.000126 |
| NH₄Cl | 1.0 |
| NaCl | 1.0 |
| MgSO₄ | 0.04 |
| K₂HPO₄ | 3.125 |
| KH₂PO₄ | 0.75 |
| | |

| **Enzyme Substrate** | |
|---|---|
| MUD | 0.20 |
| | |

| **Dissolution Aid or Solvent** | |
|---|---|
| DMSO | 0.002% |

The recovery medium described herein may be used in a sterilization monitor. In one embodiment, the sterilization monitor is a self-contained sterilization monitor that includes a container with two separate compartments. One of the compartments may contain the biological indicator. The other compartment may contain the recovery medium. In use, the sterilization monitor and the articles to be sterilized are exposed to the sterilization medium. Following sterilization, the sterilization monitor is activated so that the biological indicator comes into contact with the recovery medium sufficiently to determine whether the sterilization process is effective. The sterilization monitor may be used with any sterilization process wherein the biological indicator is exposed to the sterilization medium, for example, sterilization processes employing gaseous sterilants.

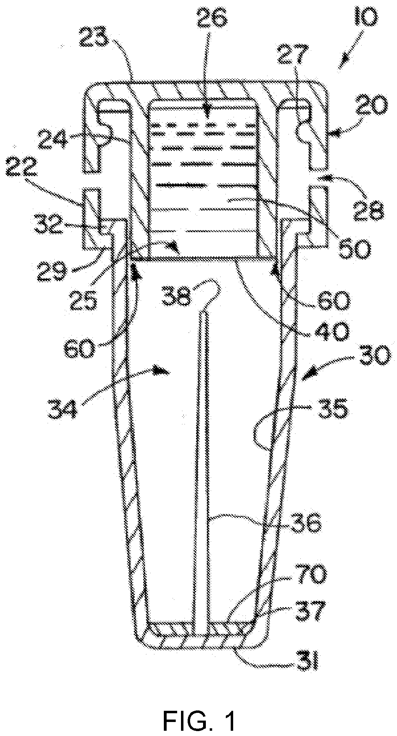

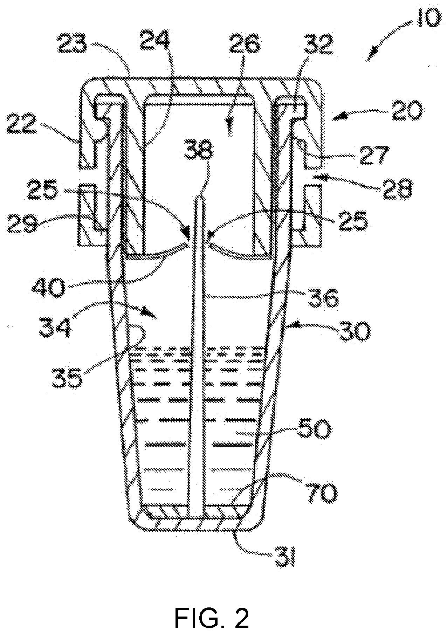

Referring to Figs. 1 and 2, a sterilization monitor 10 is disclosed. The sterilization monitor 10 includes cap 20 that is mounted on container 30. Container 30 includes a closed bottom end 31, an open upper end, and interior space 34. The cap 20 has an outer wall 22, an open lower end, and a closed upper end 23. The cap 20 also includes an inner wall 24, and an inner chamber 26. The inner chamber 26 includes an opening 25 adjacent to the bottom end of the wall 24. The inner chamber 26 contains the recovery medium 50. The cap 20 includes a breakable barrier 40 covering the opening 25 and encapsulating the recovery medium 50 within the chamber 26. The sterilization monitor 10 is configured for the cap 20 to be mounted to the container 30 in a snap-fit relationship. In other embodiments, not shown, the sterilization monitor 10 may be configured for the cap 20 to be mounted to the container 30 in a threaded relationship in which the cap 20 is engaged with the container 30 by threads and the system is activated by rotating the cap 20 with respect to the container 30, i.e., screwing the cap 20 further onto the container 30.

The container 30 includes an annular projection 32 forming a ridge or lip adjacent or near the upper end of the container 30. The cap 20 includes an annular projection 29 forming a ridge or lip adjacent the bottom of the cap 20. The cap 20 may be mounted onto the container 30 by sliding the ridge 29 of the cap over the ridge 32 of the container. The ridge 32 of the container 30 engages the ridge 29 on the cap 20 to prevent the cap 20 and container 30 from decoupling. The cap 20 and container 30 may be sized such that the ridge 32 exerts a sufficient amount of pressure against the cap 20 to prevent the cap 20 from sliding downward without applying an external downward force to the cap 20.

The container 30 includes one or more puncture members 36 which is adapted to break or puncture breakable barrier 40 when the cap 20 is moved downward, and the barrier 40 contacts the point 38 of puncture member 36. The puncture member 36 is shown as extending upwardly from bottom wall 37 of container 30. In another embodiment, not shown, puncture member 36 may extend upwardly from side wall 35, or from both the side wall 35 and bottom wall 37.

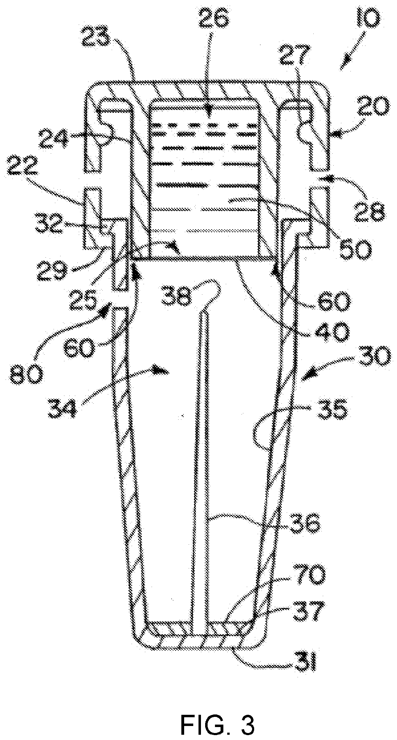

To evaluate a sterilization process, a calibrated concentration of the biological indicator is positioned within the interior 34 of the container 30. The biological indicator may be positioned directly on the walls 35 of the container or provided on a support member (e.g., support member 70) that is positioned within the container 30. The inner chamber 26 is filled with recovery medium 50. The sterilization monitor 10 is then assembled by mounting the cap 20 on the container 30. The cap 20 may be mounted by snap-fitting the cap 20 onto the container 30 as described above, or, for example, by a threaded mounting. With reference to Fig. 3, the cap 20 is mounted on the container 30 in a first, non-activated (or open) position such that the breakable barrier 40 remains intact and is not punctured by the puncture member 36.

With the sterilization monitor 10 assembled such as shown in Fig. 1, the sterilization monitor 10 then can be subjected to a sterilization process. The cap 20 has apertures 28 through which a sterilant vapor enters the sterilization monitor 10. The sterilant enters the cap through the apertures 28 (into the space between the wall 22 and the wall 24) and flows into the container 30 through a space 60 defined between the exterior surface of the inner wall 24 of the cap 20 and the inner surface of the wall 35. The sterilant vapor flows into the container 30 and contacts the biological indicator.

After the sterilization process is completed, the sterilization monitor 10 may be activated by moving the cap 20 downward toward the container 30 to a second (or closed or activated) position, which is illustrated in Fig. 2. The cap 20 is moved downward by applying a sufficient downward force or pressure on the cap 20. As the cap 20 is moved downward, the breakable barrier 40 is brought into contact with the point 38 of puncture member 36, and eventually moved into a position such that the point 38 punctures the breakable barrier 40. When the breakable barrier 40 is punctured, the recovery medium 50 drains into the interior space 34 of the container 30 and contacts the biological indicator. It may be desirable to move the cap 20 downward with a twisting motion to effect a greater or maximum opening of the breakable barrier 40 to ensure complete drainage of the inducer fluid into the container.

The inner surface of the cap 20 includes a second annular projection 27. The cap 20 may be moved downward to a position such that the upper portion of the projection 27 engages the bottom of ridge 32 of the container 30 to hold the cap 20 in a second, closed/activated position. The closed/activated position holds the cap 20 in a sealed relationship with the container 30. The sterilization monitor 10 is then incubated for a sufficient period of time to allow microorganism viability to be determined. During incubation, any viable microorganisms from the biological indicator will metabolize and grow. This metabolism releases byproducts into the recovery medium 50. The byproducts may be detected by any selected property including, for example, pH change, color change, opacity, fluorescence, and the like.

In another embodiment, the cap 20 does not include the second projection 27 to maintain the container in the closed position. The container 30 may include another annular projection or a set of detents (not shown) on the outside of the container 30 and located below the ridge 32, which projection or detents may be adapted to engage the ridge 29 on the cap to maintain the container 30 in a closed position. U.S. Patent No. 5,770,393 illustrates such a configuration, for its teachings relating to configurations of cap and container.

In another embodiment, the inner surface of the cap 20 and the outer surface of the container 30 may be threaded, and the cap 20 may be moved into and maintained in a closed position by screwing the cap 20 onto the container 30, in which the cap 20 may be threaded as shown, e.g., in U.S. Patent No. 8,173,388 B2.

The cap 20, in the embodiment illustrated in Figs. 1 and 2 is shown as having the aperture 28 to allow for the ingress of sterilant into the sterilization monitor 10. It will be appreciated, however, that the cap 20 need not be provided with such a feature. The number, size, shape, and/or location of the aperture(s) may be selected as desired, with consideration of the particular sterilant with which the sterilization indicator is to be used. For example, the location, shape, and size of the apertures in the cap 20 and/or the container 30 may be selected to provide a tortuous path for the entrance and exit of the sterilization medium between the biological indicator and the surrounding environments. In another embodiment, the space between the side wall of the cap 22 and the outer wall of the vial 30 may be sufficient to provide a torturous path without having to provide an aperture in either the cap or the vial. The tortuous path may also serve to inhibit or prevent contamination from external agents, and to make certain that an adequate amount of sterilant is available. By including the tortuous path, it is more likely that the entire load will be exposed to the sterilant thereby killing any extant microorganisms before the test organism in the sterilization monitor 10 is killed.

Apertures may be provided in the container 30 in addition to or as an alternative to providing apertures in the cap 20. Additionally, if apertures are provided in the container 30, they may be located such that the recovery medium 50 does not leak or spill out through such apertures when the sterilization monitor 10 is activated and the barrier 40 is broken.

Fig. 3 depicts sterilization monitor 10 in which an aperture 80 is formed in the sidewall 35 of the container 30 at an appropriate position, in addition to the apertures 28 in the cap 20. The aperture 80 is in the sidewall 35 of the container 30 near the top of the container 30, in the vicinity of the point 38 of the puncture member 36, to avoid leakage or spilling after activation. After activation, the aperture 80 at this location will be covered by the cap 20 in the activated position. It is noted that the sterilization monitor 10 includes the aperture 28 in the cap 20, but this may not be necessary. In one embodiment (not shown), the container 30 includes the aperture 80 and is used with a cap similar to the cap 20, but which does not include the aperture 28. Thus, an aperture can be provided either in the cap 20 or in the container 30, or in both the cap 20 and the container 30. Alternatively, no aperture may be required so long as a pathway is provided between the cap 20 and container 30 while in the unactivated state.

After the sterilization process has been completed, the cap 20 is pressed or twisted downward such that the point 38 of the puncture member 36 penetrates and breaks the breakable barrier 40 releasing the recovery medium 50 in the space 26 to mix with and incubate any of the microorganisms of the biological indicator that may have survived the sterilization process.

While the disclosed invention has been explained in relation to various detailed embodiments, it is to be understood that various modifications thereof may become apparent to those skilled in the art upon reading the specification. Therefore, it is to be understood that the invention specified herein is intended to include such modifications as may fall within the scope of the appended claims.

## Claims

1. A recovery medium for detecting the presence of microorganisms, comprising:
at least one mineral salt; and
at least one fluorogenic substrate;
wherein the recovery medium is clear in color and is substantially free of reducing sugar.

2. The recovery medium of claim 1, wherein the at least one fluorogenic substrate comprises a 4-methylumbelliferyl derivative.

3. The recovery medium of claim 2, wherein the 4-methylumbelliferyl derivative comprises 4-methylumbelliferyl α-D-glucopyranoside.

4. The recovery medium of any one of the preceding claims, wherein the fluorogenic substrate comprises a cleavable fluorophore group having a fluorescence emission in the range of 420 nm to 460 nm for an excitation wavelength in the range of 320 nm to 370 nm.

5. The recovery medium of claim 4, wherein the fluorophore comprises 4-methylumbelliferone.

6. The recovery medium of any one of the preceding claims, wherein the medium does not absorb light energy in the range of 420 nm to 460 nm.

7. The recovery medium of any one of the preceding claims, further comprising at least one amino acid and/or at least one vitamin.

8. The recovery medium of any one of the preceding claims, further comprising a non-reducing sugar, preferably wherein the non-reducing sugar is selected from among sucrose, trehalose, raffinose and melezitose.

9. The recovery medium of any one of the preceding claims, wherein the microorganisms comprise spores, preferably wherein the spores comprise spores of the *Geobacillus, Bacillus* or *Clostridia* genera.

10. A method of determining the effectiveness of a sterilization procedure, the method comprising the steps of:
placing a biological indicator containing at least one active enzyme into a sterilization chamber;
performing the sterilization procedure within the chamber wherein the biological indicator is exposed to a sterilization medium, preferably wherein the sterilization medium comprises steam, dry heat, radiation, plasma, one or more gaseous sterilants and/or one or more liquid sterilants;
contacting the biological indicator with a recovery medium comprising at least one mineral salt, and at least one fluorogenic substrate comprising a cleavable fluorophore group, wherein the recovery medium is clear in color and is substantially free of reducing sugar;
incubating the biological indicator for a period of time; and
detecting the presence or absence of a fluorescent signal emitted by the fluorophore group.

11. The method of claim 10, wherein the at least one fluorogenic substrate comprises a 4-methylumbelliferyl derivative.

12. The method of claim 11, wherein the 4-methylumbelliferyl derivative comprises 4-methylumbelliferyl α-D-glucopyranoside.

13. The method of any one of claims 10 to 12, wherein the cleavable fluorophore group has a fluorescence emission in the range of 420 nm to 460 nm for an excitation wavelength in the range of 320 nm to 370 nm.

14. The method of any one of claims 10 to 13, wherein the recovery medium does not absorb light energy in the range of 420 nm to 450 nm.

15. The method of any one of claims 10 to 14, wherein the recovery medium further comprises at least one amino acid and/or at least vitamin.

16. The method of any one of claims 10 to 15, wherein recovery medium further comprises a non-reducing sugar, preferably wherein the non-reducing sugar is selected from among sucrose, trehalose, raffinose and melezitose.

17. The method of any one of claims 10 to 16, wherein the active enzyme is associated with bacteria spores, preferably wherein the bacteria spores comprise spores of the *Geobacillus, Bacillus* or *Clostridia* genera.

18. A sterilization monitor, comprising:
a first compartment containing a biological indicator, the biological indicator containing at least one active enzyme, the first compartment being adapted to permit the biological indicator to be brought into contact with a sterilization medium during a sterilization process; and
a second compartment containing a recovery medium, the second compartment being adapted to maintain the recovery medium separate from the biological indicator during the sterilization process, and to permit the recovery medium to contact the biological indicator after the biological indicator has been exposed to the sterilization medium, wherein the recovery medium consists of the recovery medium of any one of claims 1 to 8.

## Patentansprüche

1. Rückgewinnungsmedium zum Nachweisen des Vorhandenseins von Mikroorganismen, umfassend:
mindestens ein Mineralsalz; und
mindestens ein fluorogenes Substrat;
wobei das Rückgewinnungsmedium eine klare Farbe hat und im Wesentlichen frei von reduzierendem Zucker ist.

2. Rückgewinnungsmedium nach Anspruch 1, wobei das mindestens eine fluorogene Substrat ein 4-Methylumbelliferyl-Derivat umfasst.

3. Rückgewinnungsmedium nach Anspruch 2, wobei das 4-Methylumbelliferyl-Derivat 4-Methylumbelliferyl-α-D-glucopyranosid umfasst.

4. Rückgewinnungsmedium nach einem der vorhergehenden Ansprüche, wobei das fluorogene Substrat eine spaltbare Fluorophorgruppe umfasst, die eine Fluoreszenzemission im Bereich von 420 nm bis 460 nm für eine Anregungswellenlänge im Bereich von 320 nm bis 370 nm aufweist.

5. Rückgewinnungsmedium nach Anspruch 4, wobei das Fluorophor 4-Methylumbelliferon umfasst.

6. Rückgewinnungsmedium nach einem der vorhergehenden Ansprüche, wobei das Medium keine Lichtenergie im Bereich von 420 nm bis 460 nm absorbiert.

7. Rückgewinnungsmedium nach einem der vorhergehenden Ansprüche, ferner umfassend mindestens eine Aminosäure und/oder mindestens ein Vitamin.

8. Rückgewinnungsmedium nach einem der vorhergehenden Ansprüche, ferner umfassend einen nicht-reduzierenden Zucker, wobei der nicht-reduzierende Zucker vorzugsweise aus Saccharose, Trehalose, Raffinose und Melezitose ausgewählt ist.

9. Rückgewinnungsmedium nach einem der vorhergehenden Ansprüche, wobei die Mikroorganismen Sporen umfassen, wobei die Sporen vorzugsweise Sporen der Gattungen *Geobacillus, Bacillus* oder *Clostridia* umfassen.

10. Verfahren zum Bestimmen der Wirksamkeit einer Sterilisationsprozedur, wobei das Verfahren die folgenden Schritte umfasst:
Einbringen eines biologischen Indikators, der mindestens ein aktives Enzym enthält, in eine Sterilisationskammer;
Durchführen der Sterilisationsprozedur innerhalb der Kammer, wobei der biologische Indikator einem Sterilisationsmedium ausgesetzt wird, wobei das Sterilisationsmedium vorzugsweise Dampf, Trockenhitze, Strahlung, Plasma, ein oder mehrere gasförmige Sterilisationsmittel und/oder ein oder mehrere flüssige Sterilisationsmittel umfasst;
Inkontaktbringen des biologischen Indikators mit einem Rückgewinnungsmedium, das mindestens ein Mineralsalz und mindestens ein fluorogenes Substrat umfasst, das eine spaltbare Fluorophorgruppe umfasst, wobei das Rückgewinnungsmedium eine klare Farbe hat und im Wesentlichen frei von reduzierendem Zucker ist;
Inkubieren des biologischen Indikators für einen Zeitraum; und
Nachweisen des Vorhandenseins oder Nichtvorhandenseins eines Fluoreszenzsignals, das von der Fluorophorgruppe emittiert wird.

11. Verfahren nach Anspruch 10, wobei das mindestens eine fluorogene Substrat ein 4-Methylumbelliferyl-Derivat umfasst.

12. Verfahren nach Anspruch 11, wobei das 4-Methylumbelliferyl-Derivat 4-Methylumbelliferyl-α-D-glucopyranosid umfasst.

13. Verfahren nach einem der Ansprüche 10 bis 12, wobei die spaltbare Fluorophorgruppe eine Fluoreszenzemission im Bereich von 420 nm bis 460 nm für eine Anregungswellenlänge im Bereich von 320 nm bis 370 nm aufweist.

14. Verfahren nach einem der Ansprüche 10 bis 13, wobei das Rückgewinnungsmedium keine Lichtenergie im Bereich von 420 nm bis 450 nm absorbiert.

15. Verfahren nach einem der Ansprüche 10 bis 14, wobei das Rückgewinnungsmedium ferner mindestens eine Aminosäure und/oder mindestens ein Vitamin umfasst.

16. Verfahren nach einem der Ansprüche 10 bis 15, wobei das Rückgewinnungsmedium ferner einen nicht-reduzierenden Zucker umfasst, wobei der nicht-reduzierende Zucker vorzugsweise aus Saccharose, Trehalose, Raffinose und Melezitose ausgewählt ist.

17. Verfahren nach einem der Ansprüche 10 bis 16, wobei das aktive Enzym mit Bakteriensporen assoziiert ist, wobei die Bakteriensporen vorzugsweise Sporen der Gattungen *Geobacillus, Bacillus* oder *Clostridia* umfassen.

18. Sterilisationsmonitor, umfassend:
ein erstes Fach, das einen biologischen Indikator enthält, wobei der biologische Indikator mindestens ein aktives Enzym enthält, wobei das erste Kompartiment dazu ausgestaltet ist, zu ermöglichen, dass der biologische Indikator während eines Sterilisationsprozesses mit einem Sterilisationsmedium in Kontakt gebracht wird; und
ein zweites Fach, das ein Rückgewinnungsmedium enthält, wobei das zweite Fach dazu ausgestaltet ist, das Rückgewinnungsmedium während des Sterilisationsprozesses von dem biologischen Indikator getrennt zu halten und es dem Rückgewinnungsmedium zu ermöglichen, mit dem biologischen Indikator in Kontakt zu treten, nachdem der biologische Indikator dem Sterilisationsmedium ausgesetzt wurde, wobei das Rückgewinnungsmedium aus dem Rückgewinnungsmedium nach einem der Ansprüche 1 bis 8 besteht.

## Revendications

1. Milieu de récupération pour détecter la présence de microorganismes, comprenant :
au moins un sel minéral ; et
au moins un substrat fluorogène ;
dans lequel le milieu de récupération est de couleur claire et est sensiblement exempt de sucre réducteur.

2. Milieu de récupération selon la revendication 1, dans lequel l'au moins un substrat fluorogène comprend un dérivé de 4-méthylumbelliféryle.

3. Milieu de récupération selon la revendication 2, dans lequel le dérivé de 4-méthylumbelliféryle comprend le 4-méthylumbelliféryl α-D-glucopyranoside.

4. Milieu de récupération selon l'une quelconque des revendications précédentes, dans lequel le substrat fluorogène comprend un groupe fluorophore clivable ayant une émission de fluorescence dans la plage de 420 nm à 460 nm pour une longueur d'onde d'excitation dans la plage de 320 nm à 370 nm.

5. Milieu de récupération selon la revendication 4, dans lequel le fluorophore comprend la 4-méthylumbelliférone.

6. Milieu de récupération selon l'une quelconque des revendications précédentes, dans lequel le milieu n'absorbe pas l'énergie lumineuse dans la plage de 420 nm à 460 nm.

7. Milieu de récupération selon l'une quelconque des revendications précédentes, comprenant en outre au moins un acide aminé et/ou au moins une vitamine.

8. Milieu de récupération selon l'une quelconque des revendications précédentes, comprenant en outre un sucre non réducteur, de préférence dans lequel le sucre non réducteur est choisi parmi le saccharose, le tréhalose, le raffinose et le mélézitose.

9. Milieu de récupération selon l'une quelconque des revendications précédentes, dans lequel les micro-organismes comprennent des spores, de préférence dans lequel les spores comprennent des spores des genres *Geobacillus, Bacillus* ou *Clostridia.*

10. Procédé de détermination de l'efficacité d'une procédure de stérilisation, le procédé comprenant les étapes :
de placement d'un indicateur biologique contenant au moins une enzyme active dans une chambre de stérilisation ;
d'exécution de la procédure de stérilisation à l'intérieur de la chambre dans lequel l'indicateur biologique est exposé à un milieu de stérilisation, de préférence dans lequel le milieu de stérilisation comprend de la vapeur, de la chaleur sèche, un rayonnement, du plasma, un ou plusieurs stérilisants gazeux et/ou un ou plusieurs stérilisants liquides ;
de mise en contact de l'indicateur biologique avec un milieu de récupération comprenant au moins un sel minéral, et au moins un substrat fluorogène comprenant un groupe fluorophore clivable, dans lequel le milieu de récupération est de couleur claire et sensiblement exempt de sucre réducteur ;
d'incubation de l'indicateur biologique pendant une période de temps ; et
de détection de la présence ou de l'absence d'un signal fluorescent émis par le groupe fluorophore.

11. Procédé selon la revendication 10, dans lequel l'au moins un substrat fluorogène comprend un dérivé de 4-méthylumbelliféryle.

12. Procédé selon la revendication 11, dans lequel le dérivé de 4-méthylumbelliféryle comprend le 4-méthylumbelliféryl α-D-glucopyranoside.

13. Procédé selon l'une quelconque des revendications 10 à 12, dans lequel le groupe fluorophore clivable a une émission de fluorescence dans la plage de 420 nm à 460 nm pour une longueur d'onde d'excitation dans la plage de 320 nm à 370 nm.

14. Procédé selon l'une quelconque des revendications 10 à 13, dans lequel le milieu de récupération n'absorbe pas l'énergie lumineuse dans la plage de 420 nm à 450 nm.

15. Procédé selon l'une quelconque des revendications 10 à 14, dans lequel le milieu de récupération comprend en outre au moins un acide aminé et/ou au moins une vitamine.

16. Procédé selon l'une quelconque des revendications 10 à 15, dans lequel le milieu de récupération comprend en outre un sucre non réducteur, de préférence dans lequel le sucre non réducteur est choisi parmi le saccharose, le tréhalose, le raffinose et le mélézitose.

17. Procédé selon l'une quelconque des revendications 10 à 16, dans lequel l'enzyme active est associée à des spores de bactéries, de préférence dans lequel les spores de bactéries comprennent des spores des genres *Geobacillus, Bacillus* ou *Clostridia.*

18. Moniteur de stérilisation, comprenant :
un premier compartiment contenant un indicateur biologique, l'indicateur biologique contenant au moins une enzyme active, le premier compartiment étant conçu pour permettre la mise en contact de l'indicateur biologique avec un milieu de stérilisation lors d'un processus de stérilisation ; et
un second compartiment contenant un milieu de récupération, le second compartiment étant conçu pour maintenir le milieu de récupération séparé de l'indicateur biologique pendant le processus de stérilisation, et pour permettre au milieu de récupération d'entrer en contact avec l'indicateur biologique après que l'indicateur biologique a été exposé au milieu de stérilisation, dans lequel le milieu de récupération est constitué du milieu de récupération selon l'une quelconque des revendications 1 à 8.
